(19) Europäisches Patentamt / European Patent Office / Office européen des brevets

(11) **EP 3 426 373 B1**

(12) **EUROPEAN PATENT SPECIFICATION**

(45) Date of publication and mention
of the grant of the patent:
**01.06.2022 Bulletin 2022/22**

(21) Application number: **17707927.4**

(22) Date of filing: **06.03.2017**

(51) International Patent Classification (IPC):
**B01D 29/00** *(2006.01)*       **C07C 29/132** *(2006.01)*
**C07C 29/60** *(2006.01)*

(52) Cooperative Patent Classification (CPC):
(C-Sets available)
**B01D 61/027; B01D 69/02; B01D 71/024;**
**C07C 29/132; C07C 29/60;** B01D 2313/24;
B01D 2315/14; B01D 2325/02          (Cont.)

(86) International application number:
**PCT/EP2017/055202**

(87) International publication number:
**WO 2017/153347 (14.09.2017 Gazette 2017/37)**

(54) **PROCESS FOR RECOVERING A METALLIC COMPONENT**

VERFAHREN ZUR RÜCKGEWINNUNG EINES METALLISCHEN BAUTEILS

PROCÉDÉ DE RÉCUPÉRATION D'UN COMPOSANT MÉTALLIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.03.2016 EP 16159008**

(43) Date of publication of application:
**16.01.2019 Bulletin 2019/03**

(73) Proprietor: **Shell Internationale Research
Maatschappij B.V.
2596 HR The Hague (NL)**

(72) Inventors:
• **HUIZENGA, Pieter
  1031 HW Amsterdam (NL)**
• **VAN DER HEIDE, Evert
  1031 HW Amsterdam (NL)**
• **HAAN, Johannes, Pieter
  1031 HW Amsterdam (NL)**
• **VLAANDEREN, Michel
  1031 HW Amsterdam (NL)**

(74) Representative: **Shell Legal Services IP
PO Box 384
2501 CJ The Hague (NL)**

(56) References cited:
**US-A- 5 198 007     US-A1- 2011 312 487**

(52) Cooperative Patent Classification (CPC): (Cont.)

C-Sets
**C07C 29/132;**
**C07C 29/60**

**Description**

Field of the Invention

This invention relates to a process for recovering a metallic component from a process stream and to a process for preparing glycols from a saccharide-containing feedstock.

Background of the Invention

Monoethylene glycol (MEG) and monopropylene glycol (MPG) are valuable materials with a multitude of commercial applications, e.g. as heat transfer media, antifreeze, and precursors to polymers such as polyethylene terephthalate (PET).

Said glycols are currently made on an industrial scale by hydrolysis of the corresponding alkylene oxides, which are the oxidation products of ethylene and propylene, generally produced from fossil fuels.

In recent years increased efforts have been focussed on reducing the reliance on fossil fuels as a primary resource for the provision of fuels and commodity chemicals. Carbohydrates and related biomass are seen as key renewable resources in the efforts to provide new fuels and alternative routes to desirable chemicals.

In particular, certain carbohydrates can be reacted with hydrogen in the presence of a catalyst system to generate polyols and sugar alcohols. Current methods for the conversion of saccharides to glycols revolve around a hydrogenation/retro-aldol process.

Reported processes generally require a first catalytic species to perform a retro-aldol reaction and a second catalytic species for hydrogenation of the products from the retro-aldol reaction.

Processes for the conversion of cellulose to products including MEG using nickel-promoted tungsten carbide catalysts are described in Angew. Chem. Int. Ed. 2008, 47, 8510-8513 and Catalysis Today 147 (2009), 77-85.

US 2011/0312487 A1 discloses a number of catalyst systems, including systems comprising tungstic acid, ammonium tungstate, ammonium metatungstate, phospho-tungstic acid and ammonium paratungstate as the unsupported catalyst component in conjunction with various nickel, platinum and palladium supported catalyst components.

US 2011/03046419 A1 describes a method for producing ethylene glycol from a polyhydroxy compound such as starch, hemicellulose, glucose, sucrose, fructose and fructan in the presence of catalyst comprising a first active ingredient and a second active ingredient, the first active ingredient comprising a transition metal selected from iron, cobalt, nickel, ruthenium, rhodium, palladium, iridium, and platinum, or a mixture thereof; the second active ingredient comprising a metallic state of molybdenum and/or tungsten, or a carbide, nitride, or phosphide thereof.

WO 2015028398 describes a continuous process for the conversion of a saccharide-containing feedstock into glycols. In this process the saccharide-containing feedstock is contacted in a reactor with a catalyst composition comprising at least two active catalytic components comprising, as a first active catalyst component, one or more materials selected from transition metals from groups 8, 9 or 10 or compounds thereof, with catalytic hydrogenation capabilities; and, as a second active catalyst component, one or more materials selected from tungsten, molybdenum and compounds and complexes thereof. The second active catalyst component may be present in homogeneous form.

US5198007 describes a filter useful for separating at least one contaminant from a fluid passed through the filter, which comprises a porous support composed of a fused interlocked single crystal acicular ceramic with a porous discriminating layer, such as a sintered porous alpha-alumina membrane.

Regardless of the catalytic species used, homogeneous catalyst species will be present in one or more of the process streams resulting from the conversion of saccharide-containing feedstock to glycols. Such streams will include product streams, recycle streams and bleed streams.

Homogeneous catalysts are typically recycled to the reactor as components of a process stream that is withdrawn from the reactor and partially returned to the reactor. Typically, said process stream will have been subjected to separation, e.g. distillation, in order to heave removed therefrom low boiling materials including the desired product glycols. The process stream, therefore, consists mainly of heavy hydrocarbon products that are formed in the glycol production process, e.g. the stream may comprise $C_{3+}$ sugar alcohols and carboxylic acids. A portion of this stream is removed as a bleed stream in order to prevent build-up of inerts and contaminants in the process. The bleed stream may be disposed of via flaring. This flaring typically destroys homogeneous catalysts that are present in the bleed stream, and may lead to release of metal-containing gases into the environment. Recovery of metal components from ash produced in such flaring may also be cumbersome and expensive.

The present inventors have sought to provide a process wherein metallic components (typically the homogeneous catalyst composition) may be recovered from process streams, including a bleed stream, produced in the process for the production of glycols from saccharide-containing feedstocks. Recovery of such components may enable further use of the metal and may also help to avoid release of emissions resulting from the metallic components.

Summary of the Invention

Accordingly, the present invention provides a process for recovering a metallic component from a process stream, said process comprising passing said process stream over a ceramic membrane comprising a selective layer with a pore size in the range of from at least 0.5nm to at most 10nm; applying a pressure difference across said ceramic membrane such that the pressure outside the ceramic membrane is at least 50kPa lower than the pressure inside the ceramic membrane; and, thus, providing a permeate stream which has passed through the ceramic membrane and which is depleted in the metallic component and a retentate stream enriched in the metallic component; wherein the process stream is derived from a process for the conversion of saccharide-containing feedstock into glycols, wherein the metallic component is a homogeneous catalyst composition.

The present invention also provides a process for process for preparing glycols from a saccharide-containing feedstock comprising steps of:

i) providing a saccharide-containing feedstock in a solvent and hydrogen to a reactor system, wherein the reactor system contains at least two active catalytic compositions, said active catalyst compositions comprising, as a hydrogenation catalyst composition, one or more materials selected from transition metals from groups 8, 9 or 10 or compounds thereof, with catalytic hydrogenation capabilities; and, as a retro-aldol catalyst composition, one or more homogeneous catalysts selected from tungsten, molybdenum, lanthanum, tin or compounds or complexes thereof;
ii) withdrawing a reactor product stream from the reactor system;
iii) separating the reactor product stream into at least a glycol product stream and a hydrocarbon heavies process stream, wherein the hydrocarbon heavies process stream contains a metallic component comprising a homogeneous catalyst composition; and
iv) passing at least a portion of the hydrocarbon heavies process stream over a ceramic membrane comprising a selective layer with a pore size in the range of from at least 0.5nm to at most 10nm; applying a pressure difference across said ceramic membrane such that the pressure outside the ceramic membrane is at least 50kPa lower than the pressure inside the ceramic membrane; and, thus providing a permeate stream which has passed through the ceramic membrane and which is depleted in the metallic component and a retentate stream enriched in the metallic component.

The present invention also provides a process for preparing glycols from a saccharide-containing feedstock comprising steps of:

i) contacting said saccharide-containing feedstock in a solvent and, optionally, hydrogen with a homogeneous retro-aldol catalyst composition in a first reaction zone within a reactor system, to provide an intermediate process stream comprising at least glycolaldehyde and a metallic component comprising a homogeneous catalyst composition in a solvent;
ii) passing at least a portion of said intermediate process stream over a ceramic membrane comprising a selective layer with a pore size in the range of from at least 0.5nm to at most 10nm; applying a pressure difference across said ceramic membrane such that the pressure outside the ceramic membrane is at least 50kPa lower than the pressure inside the ceramic membrane; and, thus, providing a permeate stream which has passed through the ceramic membrane and which is depleted in the metallic component and a retentate stream enriched in the metallic component; and
iii) providing said permeate stream to a second reaction zone within the reactor system and contacting it therein with hydrogen in the presence of a hydrogenation catalyst composition to provide a product stream comprising glycols.

Brief Description of the Drawings

Figures 1 to 3 are schematic diagrams representing exemplary, but non-limiting, aspects of the process of the invention.

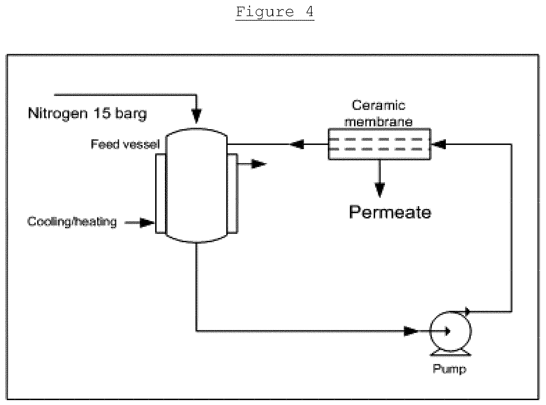

Figure 4 illustrates the process carried out in the Examples.

Detailed Description of the Invention

The present inventors have surprisingly found that a ceramic membrane may be effectively used to separate metallic components from a process stream derived from a process for the conversion of saccharides into glycols. Such a process allows the separation of metallic components from process streams at temperatures at or near the reaction temperatures used in typical processes for the conversion of saccharides into glycols. This is particularly advantageous when the process stream is a stream that is to be recycled to the process or when it is to be immediately used in another process step, as it cuts out any cooling and heating steps. The metallic components may then be recycled to the process.

Said process stream comprises a metallic component that may suitably be a metallic homogeneous catalyst composition or the degradation products that can result when such a metallic catalyst composition or a heterogeneous metallic catalyst composition degrades. The metallic component in the process stream suitably comprises one or more compound, complex or elemental material comprising tungsten, molybdenum, lanthanum or tin. Preferably, the metallic component comprises one or more compound, complex or elemental material selected from those containing tungsten or molybdenum. More preferably, the metallic component comprises one or more material selected from the list consisting of tungstic acid, molybdic acid, ammonium tungstate, ammonium metatungstate, ammonium paratungstate, tungstate compounds comprising at least one Group I or II element, metatungstate compounds comprising at least one Group I or II element, paratungstate compounds comprising at least one Group I or II element, heteropoly compounds of tungsten, heteropoly compounds of molybdenum, tungsten oxides, molybdenum oxides and combinations thereof.

The process stream typically comprises from 0.1 to 20wt% of metallic components, based upon the weight of the metal compared to the weight of the hydrocarbon product stream, preferably from 2 to 18wt%, more preferably from 5 to 15wt%.

The process stream is passed over a ceramic membrane. Said ceramic membrane suitably takes the form of a ceramic membrane disc or a tubular ceramic membrane disposed along the path that the process stream takes.

In the embodiment that the ceramic membrane is a tubular ceramic membrane, said tubular ceramic membrane is openended and is preferably disposed within a pipe, such that a portion of the pipe is formed of the tubular ceramic membrane.

The ceramic membrane comprises a selective layer has a pore size in the range of from at least 0.5nm to at most 10nm. Preferably, the pore size is in the range of from at least 0.9nm to at most 5nm.

Preferably, the material of which the selective layer of the ceramic membrane is made is selected from titania, zirconia and alumina with pore sizes in this range. Said selective layer of the ceramic membrane is typically supported on one or more further layers of oxide supports, preferably alumina. The one or more further layers will suitably have larger pore sizes than the selective layer membrane itself.

A pressure difference is applied across the ceramic membrane such that the pressure outside the ceramic membrane is at least 50kPa lower than the pressure inside the ceramic membrane, preferably at least 100kPa lower than the pressure inside the ceramic membrane. Also preferably, the pressure outside the ceramic membrane is no more than 4MPa lower than the pressure inside the ceramic membrane.

The permeate stream is depleted in the metallic component. Preferably, the permeate stream contains less than 50wt%, more preferably no more than 20wt%, even more preferably no more than 10wt%, even more preferably no more than 5wt%, even more preferably no more than 3wt%, most preferably no more than 1 wt% of the metallic component present in the process stream to be treated.

The retentate stream does not pass through the ceramic membrane and is enriched in the metallic component. Preferably, the retentate stream contains more than 50wt%, more preferably at least 80wt&, even more preferably at least 90wt%, even more preferably at least 95wt%, even more preferably at least 97wt%, most preferably at least 99wt% of the metallic component present in the process stream to be treated.

The process stream is derived from a process for the conversion of saccharide-containing feedstock into glycols. Any suitable process stream, including those set out in the description of processes for preparing glycols from a saccharide-containing feedstock below, may be used.

The present invention also provides processes for preparing glycols from a saccharide-containing feedstock. In said processes, the saccharide-containing feedstock contains one or more saccharides that are selected from the group consisting of monosaccharides, disaccharides, oligosaccharides and polysaccharides.

Saccharides, also referred to as sugars or carbohydrates, comprise monomeric, dimeric, oligomeric and polymeric aldoses, ketoses, or combinations of aldoses and ketoses, the monomeric form comprising at least one alcohol and a carbonyl function, being described by the general formula of $C_nH_{2n}O_n$ (n = 4, 5 or 6). Typical $C_4$ monosaccharides comprise erythrose and threose, typical $C_5$ saccharide monomers include xylose and arabinose and typical $C_6$ sugars comprise aldoses like glucose, mannose and galactose, while a common $C_6$ ketose is fructose. Examples of dimeric saccharides, comprising similar or different monomeric saccharides, include sucrose, maltose and cellobiose. Saccharide oligomers are present in corn syrup. Polymeric saccharides include cellulose, starch, glycogen, hemicellulose, chitin, and mixtures thereof.

If the one or more saccharides comprise oligosaccharides or polysaccharides, it is preferable that they are subjected to pre-treatment before being fed to the process in a form that can be converted in the process of the present invention. Suitable pre-treatment methods are known in the art and one or more may be selected from the group including, but not limited to, sizing, drying, grinding, hot water treatment, steam treatment, hydrolysis, pyrolysis, thermal treatment, chemical treatment, biological treatment. However, after said pre-treatment, the starting material still comprises mainly monomeric and/or oligomeric saccharides. Said saccharides are, preferably, soluble in the reaction solvent.

In one preferred embodiment of the invention, the one or more saccharides present in the saccharide-containing feedstock used in the process of the invention, after any pre-treatment, comprise saccharides selected from starch and/or hydrolysed starch. Hydrolysed starch comprises glucose, sucrose, maltose and oligomeric forms of glucose.

In another preferred embodiment of the invention, the one or more saccharides in the saccharide-containing feedstock comprise cellulose, hemi-cellulose, saccharides derived from lignocellulose, and/or sugars derived therefrom. In this embodiment, the one or more saccharides are preferably derived from softwood.

The one or more saccharides in the saccharide-containing feedstock may be derived from grains such as corn, wheat, millet, oats, rye, sorghum, barley or buckwheat, from rice, from pulses such as soybean, pea, chickpea or lentil, from bananas and/or from root vegetables such as potato, yam, sweet potato, cassava and sugar beet, or any combinations thereof. A preferred source of saccharide-containing feedstock is corn.

The one or more saccharides are suitably present as a solution, a suspension or a slurry in the solvent.

The process of the present invention is carried out in the presence of a solvent. The solvent may be water or a $C_1$ to $C_6$ alcohol or polyalcohol (including sugar alcohols), ethers, and other suitable organic compounds or mixtures thereof. Preferred $C_1$ to $C_6$ alcohols include methanol, ethanol, 1-propanol and iso-propanol. Polyalcohols of use include glycols, particularly products of the hydrogenation/ retro-aldol reaction, glycerol, erythritol, threitol, sorbitol and mixtures thereof. Preferably, the solvent comprises water.

Suitably the ratio of saccharide-containing feedstock and solvent are adjusted such that the feedstock to the reactor system contains solvent:saccharide in a ratio of between 1:1 and 5:1.

Hydrogen is required for the part of the process comprising contacting a stream with the hydrogenation catalyst composition. It may be supplied at the start of the reactor system or only to the part of the reactor system in which hydrogenation occurs, if this is a distinct part of the system, for example to the second reactor zone. The hydrogen pressure is suitably greater than 10 bar, preferably greater than 70 bar and most preferably around 100 bar. The amount of hydrogen consumed will depend upon the amount of saccharide that is provided (1 mole of glucose will react with 3 moles of hydrogen).

Within the reactor system, there may be one or more reaction zones, within which different reactions are prevalent. For example, retro-aldol reactions may be the dominant reaction in one reaction zone and hydrogenation reactions may predominate in a further reaction zone. Each reaction zone may be a distinct part of a single reactor or each reaction zone may comprise an individual reactor.

Each reaction zone may be operated with a different temperature, pressure and catalyst make-up.

Two active catalytic compositions are used in the processes of the present invention. These active catalyst compositions comprise, as a hydrogenation catalyst composition, one or more materials selected from transition metals from groups 8, 9 or 10 or compounds thereof, with catalytic hydrogenation capabilities; and, as a retro-aldol catalyst composition, one or more homogeneous catalysts selected from tungsten, molybdenum, lanthanum, tin or compounds or complexes thereof. The retro-aldol catalyst selectively cuts the saccharide molecules into smaller components.

The hydrogenation catalyst composition is suitably heterogeneous with respect to the reaction mixture. When hydrogenation catalyst composition is heterogeneous there will not be significant quantities of metal from said catalyst composition in any process stream, although it is possible that there might be very low levels of metal (e.g. up to 10ppm) that have leached from the heterogeneous catalyst that are found in any process stream.

The retro-aldol catalyst composition is a homogeneous catalyst so the hydrocarbon product stream will contain one or more metallic components that are either said homogeneous catalyst composition or degradation products resulting from the homogeneous retro-aldol catalyst composition.

Suitably, the hydrogenation catalyst composition comprises one or more of the group of metals selected from iron, cobalt, nickel, ruthenium, rhodium, palladium, iridium and platinum. This metal may be present in the elemental form or as a compound. A preferred catalyst is Raney nickel. Another possible catalyst is ruthenium dispersed on carbon.

The retro-aldol catalyst composition preferably comprises one or more homogeneous catalysts selected from tungsten or molybdenum, or compounds or complexes thereof. Most preferably, the second active catalyst comprises one or more material selected from the list consisting of tungstic acid, molybdic acid, ammonium tungstate, ammonium metatungstate, ammonium paratungstate, tungstate compounds comprising at least one Group I or II element, metatungstate compounds comprising at least one Group I or II element, paratungstate compounds comprising at least one Group I or II element, heteropoly compounds of tungsten, heteropoly compounds of molybdenum, tungsten oxides, molybdenum oxides and combinations thereof.

The temperature within the reactor system is suitably at least 130°C, preferably at least 150°C, more preferably at least 170°C, most preferably at least 190°C. The temperature within the reactor system is suitably at most 300°C, preferably at most 280°C, more preferably at most 270°C, even more preferably at most 250°C. Preferably, the reactor system is heated to a temperature within these limits before addition of any starting material and is maintained at such a temperature as the reaction proceeds.

The pressure in the reactor system is suitably at least 1 MPa, preferably at least 2 MPa, more preferably at least 3 MPa. The pressure in the reactor system is suitably at most 15 MPa, preferably at most 12 MPa, more preferably at most 10 MPa, most preferably at most 8 MPa. Preferably, the reactor system is pressurised to a pressure within these limits by addition of hydrogen before addition of any saccharide-containing feedstock or solvent and is maintained at such a pressure as the reaction proceeds through on-going addition of hydrogen.

When the reactor system includes more than one reaction zone and /or more than one reactor, the temperature and pressure in each reaction zone and/or reactor may be varied independently.

The process takes place, at least partly, in the presence of hydrogen. Preferably, the process takes place in the absence of air or oxygen. In order to achieve this, it is preferable that the atmosphere in the reactor be evacuated and replaced an inert gas, such as nitrogen, and then, where relevant, with hydrogen repeatedly, after loading of any initial reactor system contents, before the reaction starts.

Suitable reactors for use in the reactor system include stirred tank reactors, slurry reactors, ebullated bed reactors, jet flow reactors, mechanically agitated reactors, bubble columns, such as slurry bubble columns and external recycle loop reactors. The use of these reactors allows dilution of the reaction feedstock and intermediates to an extent that provides high degrees of selectivity to the desired glycol product (mainly ethylene and propylene glycols), such as by effective back-mixing.

The residence time in the reactor system is suitably at least 1 minute, preferably at least 2 minutes, more preferably at least 5 minutes. Suitably the residence time in the reactor system is no more than 5 hours, preferably no more than 2 hours, more preferably no more than 1 hour.

In embodiments of the invention wherein the process takes place in more than one reaction zone, this residence time may be split equally or disproportionally between the two or more reaction zones.

In embodiments wherein a reactor product stream is withdrawn from the reactor system, typically this stream contains solvent, hydrocarbons and homogeneous catalyst materials. The reactor product stream may then be separated into at least a glycol product stream and a hydrocarbon heavies process stream. Preferably, the reactor product stream is additionally separated into a light hydrocarbon stream and water. In a preferred separation step, the light hydrocarbon stream is first separated from the reactor product stream and then the water is removed by distillation. The glycol product stream is then separated from the hydrocarbon heavies process stream by distillation (the hydrocarbon heavies process stream is the bottom product from this distillation).

Said glycol product stream comprises as least one of monoethylene glycol (MEG), monopropylene glycol (MPG) and 1,2-butanediol (1,2-BDO). The different glycols may be collected as separate streams or as one combined stream.

A hydrocarbon heavies process stream is separated from the reactor product stream, and is, preferably, at least partially recycled back to the reactor, either directly or indirectly. The hydrocarbon heavies process stream typically contains heavy hydrocarbons and a metallic component comprising the second active catalyst component. The recycling of this stream enables reuse of the homogeneous second active catalyst component.

In one embodiment of the invention at least a portion of the hydrocarbon heavies process stream is passed over a ceramic membrane comprising a selective layer with a pore size in the range of from at least 0.5nm to at most 10nm and a pressure difference is applied across said ceramic membrane such that the pressure outside the ceramic membrane is at least 50kPa lower than the pressure inside the ceramic membrane in order to provide a permeate stream which has passed through the ceramic membrane and which is depleted in the metallic component and a retentate stream enriched in the metallic component.

In one preferred embodiment of the invention, all or substantially all of the hydrocarbon heavies process stream is passed over the ceramic membrane. In this embodiment, the permeate stream comprises in the range of from 1 to 20wt% and preferably around 10wt% of the hydrocarbon heavies process stream. This permeate stream can then be treated as a bleed stream and removed from the process. The retentate stream may then be recycled to the reactor system.

In another preferred embodiment of the invention, a portion of the hydrocarbon heavies process stream is separated as a bleed stream and it is this bleed stream that is passed over the ceramic membrane. Suitably from 1 to 20wt% and preferably around 10wt% of the hydrocarbon heavies stream is separated to provide the bleed stream. In this embodiment, the permeate stream comprises in the range of from 50 to 95wt% of the bleed stream. This permeate stream can then be removed from the process. Optionally, the retentate stream may then be recycled to the reactor system.

In the embodiment of the invention wherein the reactor system comprises two reaction zones and the intermediate process stream from the first reaction zone is passed over a ceramic membrane, the retentate stream may be recycled to the first reaction zone for re-use of catalytic material contained therein.

Detailed Description of the Drawings

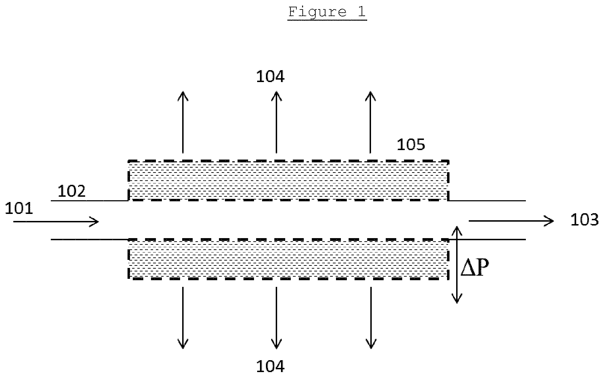

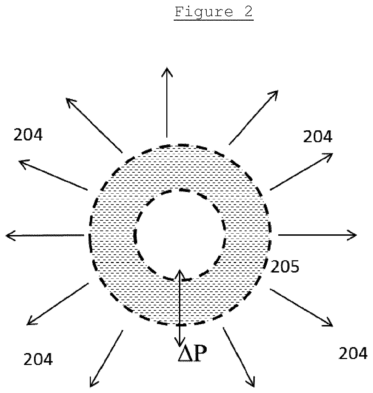

In these Figures, the first digit of each reference number refers to the Figure number (i.e. 1XX for Figure 1 and 2XX for Figure 2). The remaining digits refer to the individual features and the same features are provided with the same number in each Figure. Therefore, the same feature is numbered 104 in Figure 1 and 204 in Figure 2.

Figure 1 illustrates a non-limiting, embodiment of the present invention. A process stream 101, derived from a process for the conversion of saccharide-containing feedstock into glycols and containing a metallic component comprising a homogeneous catalyst composition, is passed through a pipe 102 and along the inside of a ceramic membrane 105 in the form of a tubular ceramic membrane. A pressure difference (ΔP) is applied across said tubular ceramic membrane. A permeate stream 104 which has passed through the ceramic membrane and which is depleted in the metallic component is provided. A retentate stream 103 enriched in the metallic component is also provided.

The same embodiment is shown in cross-section in Figure 2.

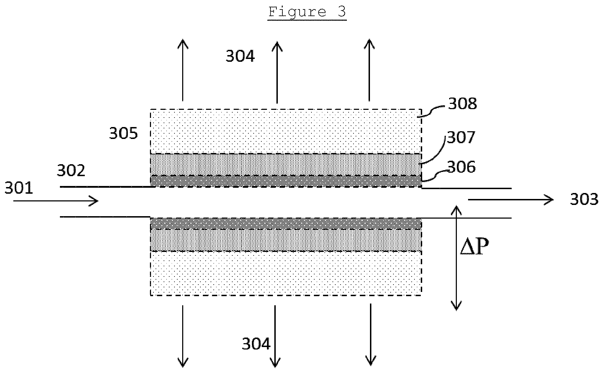

Figure 3 illustrates a ceramic membrane 305, in the form of a tubular ceramic membrane, in which the selective layer 306 of the ceramic membrane 305 is supported on further layers 307 and 308 of oxide supports, preferably alumina. The further layers 307 and 308 will suitably have larger pore sizes than the selective layer 306 of the membrane.

The invention is further illustrated by the following Examples.

Examples

Membrane testing was carried out in a small laboratory unit with a 500 ml feed vessel. A cross-flow of 80 l/hr across the ceramic membrane surface of 0.00187 $m^2$ is maintained by means of a pump.

The feed vessel is heated by means of an external oil bath and by proper insulation the desired temperature can be achieved. The unit can be pressurized at the feed side of the membrane by means of 15 barg (16 bar absolute) nitrogen. The mass of the collected permeate is recorded against time and accordingly the permeate mass flow in kg/hr is calculated.

The Trans Membrane Pressure (TMP) is the driving force for transport through the pores of the membrane and is defined as the average pressure difference between the Feed/Retentate side and the Permeate side of the membrane.

$$TMP = (Pin + Pout)/2 - Ppermeate$$

Pin = Pressure at the membrane entrance (feed in)
Pout = Pressure at the membrane outlet (retentate out)
Ppermeate = Pressure at the membrane permeate side

The membrane flux ($kg.m^{-2}.hr^{-1}$) is calculated from the measured permeate flow in kg/hr divided by the used membrane area. The membrane permeability ($kg.m^{-2}.hr^{-1}.bar^{-1}$) is defined as the flux divided by the TMP. The rejection is a measure for a component (Tungsten) which does not pass the membrane and is retained by the membrane. The rejection is calculated from the Tungsten concentrations in the respective permeate and retentate streams, i.e. the Tungsten concentrations in the final total permeate and total retentate volumes.

$$Rejection = (1-[Wpermeate]/[Wretentate]) *100\%$$

[Wpermeate]=Tungsten concentration in total permeate (mg/kg)
[Wretentate]=Tungsten concentration in total retentate (mg/kg)
The permeate recovery relates the amount of permeate collected and the amount of feed used in the experiment.

$$Permeate\ recovery = ((kg\ of\ permeate\ collected)\ /\ (kg\ of\ feed))*100\%.$$

Example 1

In 400 grams of a 1:1 (weight ratio) glycerol/water mixture 3.38 gram of sodium metatungstate.monohydrate ($Na_6W_{12}O_9.H_2O$) was dissolved. The feed vessel of the membrane unit was filled with 393.8 gram of this solution. A tubular 5 nm pore size membrane, Titania selective layer (ex Inopor, Germany) was installed in the cross-flow unit. The circulation pump was started and when the liquid reached a temperature of 90 °C a Trans Membrane Pressure of 15.2 bar was applied by means of pressurizing the feed side of the system with nitrogen gas. During the experiment the temperature was increased to 94°C. In approximately 3 hours, 212.9 gram of permeate was collected and after cooling down of the unit, 160.7 gram retentate could be withdrawn. From this a mass loss of 20.2 gram was calculated. A permeate mass flow of 0.070kg/h was calculated. A summary of the results is shown in Table 1.

Example 2

In 404 grams of a 1:1 (weight ratio) glycerol/water mixture 3.38 gram of sodium metatungstate.monohydrate ($Na_6W_{12}O_9.H_2O$) was dissolved. The feed vessel of the membrane unit was filled with 394.7 gram of this solution. A tubular 3 nm pore size membrane, Zirconia selective layer (ex Inopor, Germany) was installed in the cross-flow unit. The circulation pump was started and when the liquid reached a temperature of 90 °C a Trans Membrane Pressure of 15.2 bar was applied by means of pressurizing the feed side of the system with nitrogen gas. During experiment the temperature increased to 93 °C. In approximately 2 hrs 307.9 gram of permeate was collected and after cooling down of the unit, 59.9 gram retentate could be withdrawn. From this a mass loss of 26.9 gram was calculated. A permeate mass flow of 0.158 kg/hr was calculated. A summary of the results is shown in Table 1.

Table 1

| | | Example 1 | Example 2 |
|---|---|---|---|
| | | | |
| Membrane | | 5 nm $TiO_2$ (ex Inopor) | 3 nm $ZrO_2$ (ex Inopor) |
| Temperature | °C | 90-94 | 90-93 |
| Trans Membrane Pressure | bar | 15.2 | 15.2 |
| Polar liquid: glycerol/water | w: w | 1:1 | 1:1 |
| Membrane area | $m^2$ | 0.00187 | 0.00187 |
| Membrane flux | $kg.m^{-2}.hr^{-1}$ | 37.4 | 84.5 |
| Permeability | $kg.m^{-2}.hr^{-1}.bar^{-1}$ | 2.5 | 5.6 |
| Permeate recovery | %wt. | 54 | 78 |
| W content, feed | mg/kg | 6197 | 6065 |
| W content, retentate | mg/kg | 7896 | 26186 |
| W content, permeate | mg/kg | 4778 | 810 |
| W rejection | %wt. | 39.5 | 96.9 |
| W in permeate/W in Retentate (mass ratio) | %wt. | 41.6 | 10.4 |

The examples indicate metal recovery using both membranes. However, there is a clear difference between the two membranes under comparable conditions. The 3 nm pores size Zirconia membrane is much more selective in rejecting the Tungsten containing molecule. In this case a high rejection of Tungsten is obtained: 96.9 %.

**Claims**

1.   A process for recovering a metallic component from a process stream, said process comprising passing said process stream over a ceramic membrane comprising a selective layer with a pore size in the range of from at least 0.5nm to at most 10nm; applying a pressure difference across said ceramic membrane such that the pressure outside the ceramic membrane is at least 50kPa lower than the pressure inside the ceramic membrane; and, thus, providing a permeate stream which has passed through the ceramic membrane and which is depleted in the metallic component and a retentate stream enriched in the metallic component; wherein the process stream is derived from a process for the conversion of saccharide-containing feedstock into glycols, wherein the metallic component is a homogeneous catalyst composition.

2.   A process for preparing glycols from a saccharide-containing feedstock comprising steps of:

   i) providing a saccharide-containing feedstock in a solvent and hydrogen to a reactor system, wherein the reactor system contains at least two active catalytic compositions, said active catalyst compositions comprising, as a hydrogenation catalyst composition, one or more materials selected from transition metals from groups 8, 9 or 10 or compounds thereof, with catalytic hydrogenation capabilities; and, as a retro-aldol catalyst composition, one or more homogeneous catalysts selected from tungsten, molybdenum, lanthanum, tin or compounds or complexes thereof;

ii) withdrawing a reactor product stream from the reactor system;

iii) separating the reactor product stream into at least a glycol product stream and a hydrocarbon heavies process stream, wherein the hydrocarbon heavies process stream contains a metallic component comprising a homogeneous catalyst composition; and

iv) passing at least a portion of the hydrocarbon heavies process stream over a ceramic membrane comprising a selective layer with a pore size in the range of from at least 0.5nm to at most 10nm; applying a pressure difference across said ceramic membrane such that the pressure outside the ceramic membrane is at least 50kPa lower than the pressure inside the ceramic membrane; and, thus providing a permeate stream which has passed through the ceramic membrane and which is depleted in the metallic component and a retentate stream enriched in the metallic component.

3. A process for process for preparing glycols from a saccharide-containing feedstock comprising steps of:

i) contacting said saccharide-containing feedstock in a solvent and, optionally, hydrogen with a homogeneous retro-aldol catalyst composition in a first reaction zone within a reactor system, to provide an intermediate process stream comprising at least glycolaldehyde and a metallic component comprising the homogensous retro-aldol catalyst composition in a solvent;

ii) passing at least a portion of said intermediate process stream over a ceramic membrane comprising a selective layer with a pore size in the range of from at least 0.5nm to at most 10nm; applying a pressure difference across said ceramic membrane such that the pressure outside the ceramic membrane is at least 50kPa lower than the pressure inside the ceramic membrane; and, thus, providing a permeate stream which has passed through the ceramic membrane and which is depleted in the metallic component and a retentate stream enriched in the metallic component; and

iii) providing said permeate stream to a second reaction zone within the reactor system and contacting it therein with hydrogen in the presence of a hydrogenation catalyst composition to provide a product stream comprising glycols.

4. A process as claimed in claim 2, wherein all or substantially all of the hydrocarbon heavies process stream is passed over the ceramic membrane and the permeate stream comprises in the range of from 1 to 20wt% of the hydrocarbon heavies process stream.

5. A process as claimed in claim 2, wherein from 1 to 20wt% of the hydrocarbon heavies process stream is passed over the ceramic membrane having been separated as a bleed stream and the permeate stream comprises in the range of from 50 to 95wt% of said bleed stream.

6. A process as claimed in any one of claims 2, 4 and 5, wherein the retentate stream is recycled to the reactor system.

7. A process as claimed in any one of claims 1 to 6, wherein the ceramic membrane is in the form of a tubular ceramic membrane or a ceramic membrane disc.

8. A process as claimed in any one of claims 1 to 7, wherein the selective layer of the ceramic membrane is made from a material selected from titania, zirconia, alumina and mixtures thereof.

9. A process as claimed in any one of claims 1 to 8, wherein the selective layer of the ceramic membrane is supported on one or more further layers of oxide support having larger pore sizes than the selective layer.

10. A process as claimed in any one of claims 1 to 9, wherein the permeate stream contains no more than 3wt% of the metallic component present in the process stream.

**Patentansprüche**

1. Vorgang zum Zurückgewinnen einer metallischen Komponente aus einem Vorgangsstrom, wobei der Vorgang ein Führen des Vorgangsstroms über eine Keramikmembran umfasst, die eine Selektionsschicht mit einer Porengröße in dem Bereich von wenigstens 0,5 nm bis höchstens 10 nm umfasst; Aufbringen eines Druckunterschieds über die Keramikmembran hinweg, derart, dass der Druck außerhalb der Keramikmembran wenigstens 50 kPa niedriger als der Druck innerhalb der Keramikmembran ist; und somit Bereitstellen eines Permeatstroms, der durch die Keramikmembran geführt wurde und der in der metallischen Komponente verarmt ist, und eines Retentatstroms, der in der metallischen Komponente angereichert ist; wobei der Vorgangsstrom aus einem Vorgang für die Umwandlung von saccharidhaltigem Ausgangsmaterial in Glykole abstammt, wobei die metallische Komponente eine homogene Katalysatorzusammensetzung ist.

2.  Vorgang zum Herstellen von Glykolen aus einem saccharidhaltigen Ausgangsmaterial, der folgende Schritte umfasst:

   i) Bereitstellen eines saccharidhaltigen Ausgangsmaterials in einem Lösungsmittel und von Wasserstoff an ein Reaktorsystem, wobei das Reaktorsystem wenigstens zwei aktive katalytische Zusammensetzungen enthält, wobei die aktiven Katalysatorzusammensetzungen als eine Hydrierungskatalysatorzusammensetzung ein oder mehrere Materialien, die aus Übergangsmetallen aus Gruppen 8, 9 oder 10 oder Verbindungen davon mit katalytischen Hydrierungsfähigkeiten ausgewählt sind, und als eine Retro-Aldol-Katalysatorzusammensetzung einen oder mehrere homogene Katalysatoren, die aus Wolfram, Molybdän, Lanthan, Zinn oder Verbindungen oder Komplexen davon ausgewählt sind, umfassen;
   ii) Entnehmen eines Reaktorerzeugnisstroms aus dem Reaktorsystem;
   iii) Trennen des Reaktorerzeugnisstroms in wenigstens einen Glykolerzeugnisstrom und einen Vorgangsstrom aus schweren Kohlenwasserstoffen, wobei der Vorgangsstrom aus schweren Kohlenwasserstoffen eine metallische Komponente enthält, die eine homogene Katalysatorzusammensetzung umfasst; und
   iv) Führen wenigstens eines Abschnitts des Vorgangsstroms aus schweren Kohlenwasserstoffen über eine Keramikmembran, die eine Selektionsschicht mit einer Porengröße in dem Bereich von wenigstens 0,5 nm bis höchstens 10 nm umfasst; Aufbringen eines Druckunterschieds über die Keramikmembran hinweg, derart, dass der Druck außerhalb der Keramikmembran wenigstens 50 kPa niedriger als der Druck innerhalb der Keramikmembran ist; und somit Bereitstellen eines Permeatstroms, der durch die Keramikmembran geführt wurde und der in der metallischen Komponente verarmt ist, und eines Retentatstroms, der in der metallischen Komponente angereichert ist.

3.  Vorgang für den Vorgang zum Herstellen von Glykolen aus einem saccharidhaltigen Ausgangsmaterial, der folgende Schritte umfasst:

   i) Inberührungbringen des saccharidhaltigen Ausgangsmaterials in einem Lösungsmittel und optional Wasserstoff mit einer homogenen Retro-Aldol-Katalysatorzusammensetzung in einer ersten Reaktionszone innerhalb eines Reaktorsystems, um einen intermediären Vorgangsstrom bereitzustellen, der wenigstens Glykolaldehyd und eine metallische Komponente umfasst, die die homogenen Retro-Aldol-Katalysatorzusammensetzung in einem Lösungsmittel umfasst;
   ii) Führen wenigstens eines Abschnitts des intermediären Vorgangsstroms über eine Keramikmembran, die eine Selektionsschicht mit einer Porengröße in dem Bereich von wenigstens 0,5 nm bis höchstens 10 nm umfasst; Aufbringen eines Druckunterschieds über die Keramikmembran hinweg, derart, dass der Druck außerhalb der Keramikmembran wenigstens 50 kPa niedriger als der Druck innerhalb der Keramikmembran ist; und somit Bereitstellen eines Permeatstroms, der durch die Keramikmembran geführt wurde und der in der metallischen Komponente verarmt ist, und eines Retentatstroms, der in der metallischen Komponente angereichert ist; und
   iii) Bereitstellen des Permeatstroms an eine zweite Reaktionszone innerhalb des Reaktorsystems und Inberührungbringen desselben darin mit Wasserstoff in der Gegenwart einer Hydrierungskatalysatorzusammensetzung, um einen Erzeugnisstrom bereitzustellen, der Glykole umfasst.

4.  Vorgang nach Anspruch 2, wobei der gesamte oder im Wesentlichen der gesamte Vorgangsstrom aus schweren Kohlenwasserstoffen über die Keramikmembran geführt wird und der Permeatstrom in dem Bereich von 1 bis 20 Gew.-% des Vorgangsstroms aus schweren Kohlenwasserstoffen umfasst.

5.  Vorgang nach Anspruch 2, wobei von 1 bis 20 Gew.-% des Vorgangsstroms aus schweren Kohlenwasserstoffen über die Keramikmembran geführt werden, nachdem sie als ein Ausschleusestrom getrennt wurden, und der Permeatstrom in dem Bereich von 50 bis 95 Gew.-% des Ausschleusestroms umfasst.

6.  Vorgang nach einem der Ansprüche 2, 4 und 5, wobei der Retentatstrom in das Reaktorsystem rückgeführt wird.

7.  Vorgang nach einem der Ansprüche 1 bis 6, wobei die Keramikmembran in der Form einer röhrenförmigen Keramikmembran oder einer Keramikmembranscheibe vorliegt.

8. Vorgang nach einem der Ansprüche 1 bis 7, wobei die Selektionsschicht der Keramikmembran aus einem Material geschaffen ist, das aus Titanoxid, Zirkonoxid, Aluminiumoxid und Mischungen davon ausgewählt ist.

9. Vorgang nach einem der Ansprüche 1 bis 8, wobei die Selektionsschicht der Keramikmembran auf einer oder mehreren weiteren Schichten aus Oxidträger, die größere Poren als die Selektionsschicht aufweisen, getragen wird.

10. Vorgang nach einem der Ansprüche 1 bis 9, wobei der Permeatstrom nicht mehr als 3 Gew.-% der in dem Vorgangsstrom vorhandenen metallischen Komponente enthält.

## Revendications

1. Procédé de récupération d'un composant métallique à partir d'un flux de traitement, ledit procédé comprenant le passage dudit flux de traitement sur une membrane céramique comprenant une couche sélective avec une taille de pore dans la plage d'au moins 0,5 nm jusqu'à, au maximum, 10 nm ; l'application d'une différence de pression à travers ladite membrane céramique de telle sorte que la pression à l'extérieur de la membrane céramique soit plus faible d'au moins 50 kPa que la pression à l'intérieur de la membrane céramique ; et, ainsi, la production d'un flux de perméat qui a traversé la membrane céramique et qui est appauvri en composant métallique et d'un flux de rétentat enrichi en composant métallique ; dans lequel le flux de traitement est dérivé d'un procédé de conversion de matière première contenant des saccharides en glycols, dans lequel le composant métallique est une composition de catalyseur homogène.

2. Procédé de préparation de glycols à partir d'une matière première contenant des saccharides, comprenant les étapes consistant à :

   i) fournir une matière première contenant des saccharides dans un solvant et de l'hydrogène à un système réacteur, le système réacteur contenant au moins deux compositions de catalyseur actives, lesdits compositions de catalyseur actives comprenant, comme composition de catalyseur d'hydrogénation, un ou plusieurs matériaux choisis parmi les métaux de transition des groupes 8, 9 ou 10 ou des composés de ceux-ci, présentant des capacités d'hydrogénation catalytique ; et, comme composition de catalyseur de rétro-aldol, un ou plusieurs catalyseurs homogènes choisis parmi le tungstène, le molybdène, le lanthane, l'étain ou des composés ou des complexes de ceux-ci ;
   ii) extraire un flux de produit de réacteur du système réacteur ;
   iii) séparer le flux de produit du réacteur en au moins un flux de produit de glycol et un flux de traitement d'hydrocarbures lourds, dans lequel le flux de traitement d'hydrocarbures lourds contient un composant métallique comprenant une composition de catalyseur homogène ; et
   iv) faire passer au moins une partie du flux de traitement d'hydrocarbures lourds sur une membrane céramique comprenant une couche sélective avec une taille de pore dans la plage d'au moins 0,5 nm jusqu'à, au maximum, 10 nm ; appliquer une différence de pression à travers ladite membrane céramique de telle sorte que la pression à l'extérieur de la membrane céramique est plus faible d'au moins 50 kPa que la pression à l'intérieur de la membrane céramique ; et, ainsi, fournir un flux de perméat qui a traversé la membrane céramique et qui est appauvri en composant métallique et un flux de rétentat enrichi en composant métallique.

3. Procédé de préparation de glycols à partir d'une matière première contenant des saccharides, comprenant les étapes consistant à :

   i) mettre en contact ladite matière première contenant des saccharides dans un solvant et, éventuellement, de l'hydrogène avec une composition de catalyseur de rétro-aldol homogène dans une première zone de réaction à l'intérieur d'un système de réacteur, pour fournir un flux de traitement intermédiaire comprenant au moins du glycolaldéhyde et un composant métallique comprenant la composition de catalyseur de rétro-aldol homogène dans un solvant ;
   ii) faire passer au moins une partie dudit flux de traitement intermédiaire sur une membrane céramique comprenant une couche sélective avec une taille de pore dans la plage d'au moins 0,5 nm jusqu'à, au maximum, 10 nm ; appliquer une différence de pression à travers ladite membrane céramique de telle sorte que la pression à l'extérieur de la membrane céramique est plus faible d'au moins 50 kPa que la pression à l'intérieur de la membrane céramique ; et, ainsi, fournir un flux de perméat qui a traversé la membrane céramique et qui est appauvri en composant métallique et un flux de rétentat enrichi en composant métallique ; et

iii) fournir ledit flux de perméat à une seconde zone de réaction à l'intérieur du système de réacteur et le mettre en contact dans celle-ci avec de l'hydrogène en présence d'une composition de catalyseur d'hydrogénation pour fournir un flux de produit comprenant des glycols.

4. Procédé selon la revendication 2, dans lequel la totalité ou la quasi-totalité du flux de traitement d'hydrocarbures lourds passe sur la membrane céramique et le flux de perméat comprend dans la plage de 1 à 20 % en poids du flux de traitement d'hydrocarbures lourds.

5. Procédé selon la revendication 2, dans lequel de 1 à 20 % en poids du flux de traitement d'hydrocarbures lourds sont passés sur la membrane céramique ayant été séparée en tant que flux de purge et le flux de perméat comprend dans la plage allant de 50 à 95 % en poids dudit flux de purge.

6. Procédé selon l'une quelconque des revendications 2, 4 et 5, dans lequel le flux de rétentat est recyclé vers le système de réacteur.

7. Procédé selon l'une quelconque des revendications 1 à 6, dans lequel la membrane céramique se présente sous la forme d'une membrane céramique tubulaire ou d'un disque de membrane céramique.

8. Procédé selon l'une quelconque des revendications 1 à 7, dans lequel la couche sélective de la membrane céramique est constituée d'un matériau choisi parmi l'oxyde de titane, la zircone, l'alumine et leurs mélanges.

9. Procédé selon l'une quelconque des revendications 1 à 8, dans lequel la couche sélective de la membrane céramique est supportée sur une ou plusieurs autres couches de support d'oxyde ayant des tailles de pores plus grandes que la couche sélective.

10. Procédé selon l'une quelconque des revendications 1 à 9, dans lequel le flux de perméat ne contient pas plus de 3 % en poids du composant métallique présent dans le flux de traitement.

Figure 1

Figure 2

Figure 3

Figure 4

Ceramic membrane

Permeate

Pump

Nitrogen 15 barg

Feed vessel

Cooling/heating

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20110312487 A1
- US 201103046419 A1
- WO 2015028398 A
- US 5198007 A

### Non-patent literature cited in the description

- *Angew. Chem. Int. Ed.,* 2008, vol. 47, 8510-8513
- *Catalysis Today,* 2009, vol. 147, 77-85